# EUROPEAN PATENT APPLICATION

(11) **EP 3 611 639 A1**
(43) Date of publication of application: **19.02.2020**
(21) Application number: 18784647.2
(22) Date of filing: 10.04.2018
(51) Int. Cl.: G06F 17/50

(54) **METHOD FOR CUSTOMIZING PRODUCT ACCORDING TO PRESSURE DETECTION RESULTS**

(30) Priority: 11.04.2017 CN 201710232622
(71) Applicant: Qingyuan Global Technology Services Ltd., Qingyuan City, Guangdong 51180 (CN)
(72) Inventor: LUH, Yih-Ping, Taipei City Taiwan 104 (TW)
(74) Representative: dompatent von Kreisler Selting Werner - Partnerschaft von Patent- und Rechtsanwälten mbB
(86) International application number: PCT/CN2018/082454
(87) International publication number: WO 2018/188577

(57) **Abstract**

The present disclosure provides a product customization method based on a result of pressure detection, wherein, an operation chip of a capacitance pressure detection insole receives a plurality of capacitance variations from a plurality of capacitance sensing nodes and obtains pressure data of corresponding to detecting points of the user's foot based on a plurality of capacitance variations, so that sport physiological status information is decided; the sport physiological status information is received from the operational chip by a data reading device and the sport physiological status information is transmitted to a cloud database or a remote computing device through a network; corrective recommendation information is determined from the sport physiological information, afoot correction insole model corresponding to the corrective recommendation information is generated based on the corrective recommendation information; an insole corresponding to the foot correction insole model is produced and provided based on the correction insole model.

## Description

### BACKGROUND OF THE INVENTION

### 1. Field of the Invention

The present disclosure relates to a product customization method; in particular, the present disclosure relates to a product customization method based on the result of detecting insoles pressure.

### 2. Description of the Prior Art

Along with constant evolution of technology, through different designs in thickness and materials, insoles disposed in shoes may provide users with different functions such as height increase and damping in addition to the functions of traditional insoles.

However, due to different distribution profiles of pressure points on the soles of every users, if insoles are produced by mass production as what are available on the current market, it is impossible to satisfy the actual demands of every users. As a result, many users feel uncomfortable when they walk or exercise wearing shoes. With the mass-produced insoles, it is also impossible to correct any foot problems the user has, resultling in unimproved or even aggravated foot problems.

### SUMMARY OF THE INVENTION

In view of this, the present disclosure provides a product customization method based on the result of detecting status of the foot, so that the problems encountered by the current technology may be solved effectively.

A detailed embodiment according to the present disclosure is a product customization method of insoles. In the present embodiment, the method includes: An operation chip in a capacitance pressure detection insole receives a plurality of capacitance variations from a plurality of capacitance sensing nodes to obtain pressure data corresponding to the detection points of a user's foot based on the plurality of capacitance variations and determine sport physiological status information; a data reading device receives the sport physiological status information from the operational chip and transmits the sport physiological status information to a cloud database or a remote computing device through the internet; corrective recommendation information is determined from the sport physiological status information and a foot correction insole model is generated based on the corrective recommendation information; an insole corresponding to the foot correction insole model is produced and provided based on the foot correction insole model.

In an embodiment, the step of determining the sport physiological status information further includes: respectively sensing capacitance variations corresponding to the plurality of detection points of the foot by the capacitance sensing nodes when the capacitance pressure detection insole receives a pressure applied by a user's foot.

In an embodiment, the method further includes: receiving the capacitance variations from the plurality of capacitance sensing nodes to obtain pressure distribution information corresponding to the detection points of the foot based on the capacitance variations to determine sport physiological status information of the user's foot by the operational chip.

In an embodiment, the operational chip includes a receiving unit and a numerical conversion unit. The step of obtaining the pressure data corresponding to the detecting points of the user's foot based on the plurality of capacitance variations further includes: receiving M number of original detection data by the receiving unit, wherein M is a positive integer; and conducting a numerical conversion process to the M number of original detection data by the numerical conversion unit to generate K number of numerical converted data, wherein K is a positive integer and smaller than M, so that the data amount of the K number of numerical converted data is smaller than that of the M number of original detection data.

In an embodiment, the method further includes: using a numerical conversion mechanism to conduct a numerical conversion process to the M number of original detection data by the numerical conversion unit.

In an embodiment, the numerical conversion mechanism is a Fourier transform mechanism or a Laplace transform mechanism.

In an embodiment, the method further includes: outputting the K number of numerical conversion data to the internet; and connecting to the cloud database or the remote computing device through the internet.

In an embodiment, the method further includes: determining the sport physiological status information of the user's foot according to the K number of numerical converted data and transmitting the sport physiological status information to the internet.

In an embodiment, the operational chip is a Bluetooth chip embedded in the capacitance pressure insole. The step of obtaining the sport physiological status information from the operational chip by the reading device further includes: when the operational chip is located near the data reading device, the operational chip and the data reading device communicate with each other by a Bluetooth communication protocol.

In an embodiment, the step of determining corrective recommendation information from the sport physiological status information further includes: comparing the sport physiological status information with an average value of normal feet; analyzing the differences between the sport physiological status information and the average value based on the comparison result to generate corrective recommendation information.

In an embodiment, the method further includes: determining a distribution profile of pressure applied on the insole by the user's foot based on the sport physiological status information and comparing the distribution profile of pressure with the average value of normal feet.

In an embodiment, the data reading device includes a device including internet of things, Bluetooth, or wireless network.

### BRIEF DESCRIPTION OF THE DRAWINGS

In the attached FIGs, the digital identifier labeled in the leftmost means the order firstly appeared on the attached FIGs. When the same symbol is described in the different paragraphs and attachment FIGs, it means that the same element is described.
FIG. 1 is a diagram of the capacitance pressure detection insole 2 in the present disclosure disposed on shoe 1.
FIG. 2 is a diagram of the capacitance pressure detection insole 2 having a plurality of capacitance sensing nodes N in the present disclosure.
FIG. 3 is a diagram of a plurality of capacitance sensing nodes N in the capacitance pressure detection insole 2, located under a thermoplastic polyester elastomer (TPEE) layer TP and an operational chip CH embedded in the capacitance pressure detection insole 2 in the present disclosure.
FIG. 4 is a diagram of the operational chip CH receiving the plurality of capacitance variations corresponding to a plurality of detection points P1, P2, P3 ,...of the foot FT respectively detected by the plurality of capacitance sensing nodes N1, N2, N3,...and connecting to the cloud database DB or the Mobile Broadcast MB through the internet NET when the capacitance pressure detection insole 2 receives pressure applied by the user's foot FT.
FIG. 5 is a diagram of a capacitance line L1 and a conductive line L2 combined with each other.
FIG. 6 is a block diagram of a function of the data processing unit in the present disclosure.
FIG. 7 is a diagram of an embodiment of a user excising.
FIG. 8 is a flowchart of a product customization method in the present disclosure.

### DETAILED DESCRIPTION OF THE PREFERRED EMBODIMENT

A detailed embodiment according to the present disclosure is a data processing unit. In the present embodiment, the data processing unit may be a micro processing unit, a Bluetooth chip or internet of things (IOT) chip, and may be embedded in a capacitance pressure detection insole, but not limited thereto. When the data processing unit receives M number of original detection data, the data processing unit will conduct a numerical conversion process to the M number of original detection data to generate K number of numerical converted data, wherein both M and K are integers and K is smaller than M, so that the data amount of the K number of numerical converted data will be smaller than that of the M number of original detection data, so that the effect of decreasing data amount may be achieved.

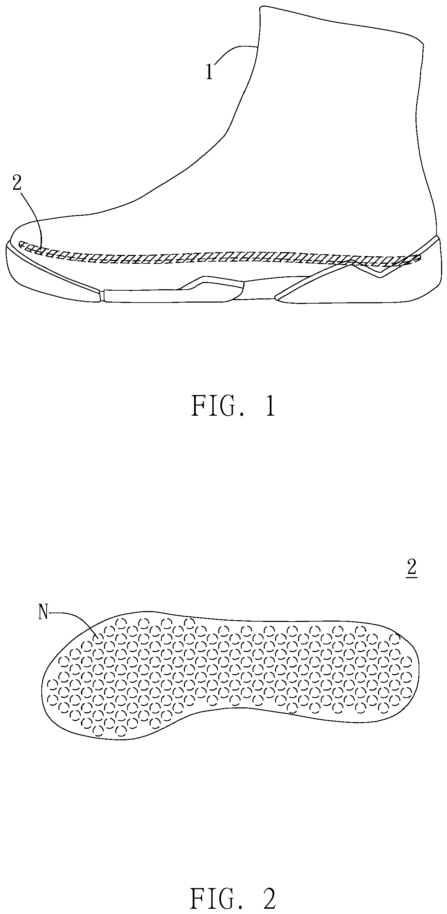

If the data processing unit is embedded in an operational chip in the capacitance pressure detection insole, then the M number of original detection data received by the data processing unit may be the M number of capacitance variations respectively detected by the M number of capacitance sensing nodes of the capacitance pressure detection insole, and the M number of capacitance variations respectively corresponds to the M number of detection points of a user's foot. Please refer to FIG. 1 and FIG. 2. FIG. 1 describes the capacitance pressure detection insole 2 disposed on shoe 1, and FIG. 2 describes the capacitance pressure detection insole 2 having a plurality of capacitance sensing nodes N.

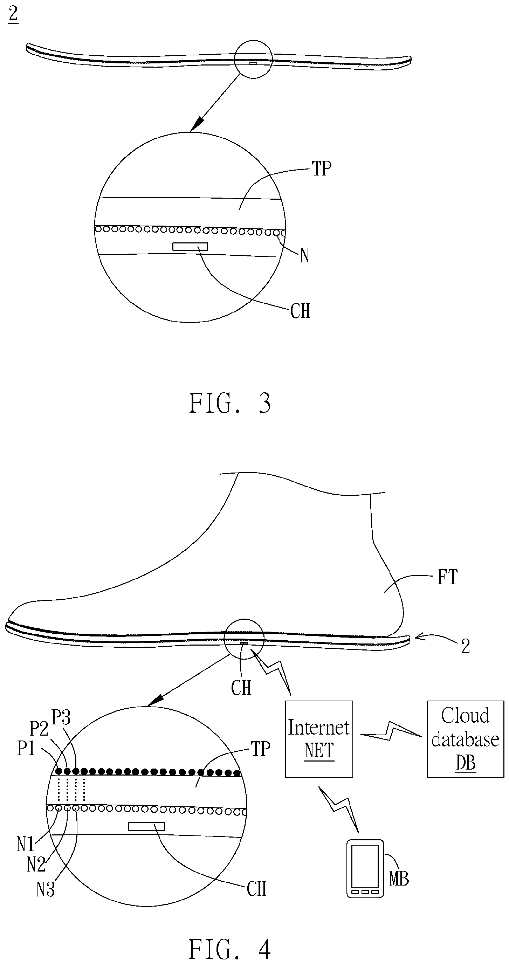

Then, please refer to FIG. 3 and FIG. 4. FIG. 3 is a diagram of the capacitance pressure detection insole 2 having not received pressure applied by foot of the user yet; FIG. 4 is a diagram of the capacitance pressure detection insole 2 having received pressure applied by foot FT.

As shown in FIG. 3 and FIG. 4, the capacitance pressure detection insole 2 includes a thermoplastic polyester elastomer (TPEE) layer TP, the plurality of capacitance sensing nodes N, and an operational chip CH. Wherein, the plurality of capacitance sensing nodes N are under the thermoplastic polyester elastomer (TPEE) layer TP, and the operational chip CH is embedded in the capacitance pressure detection insole 2.

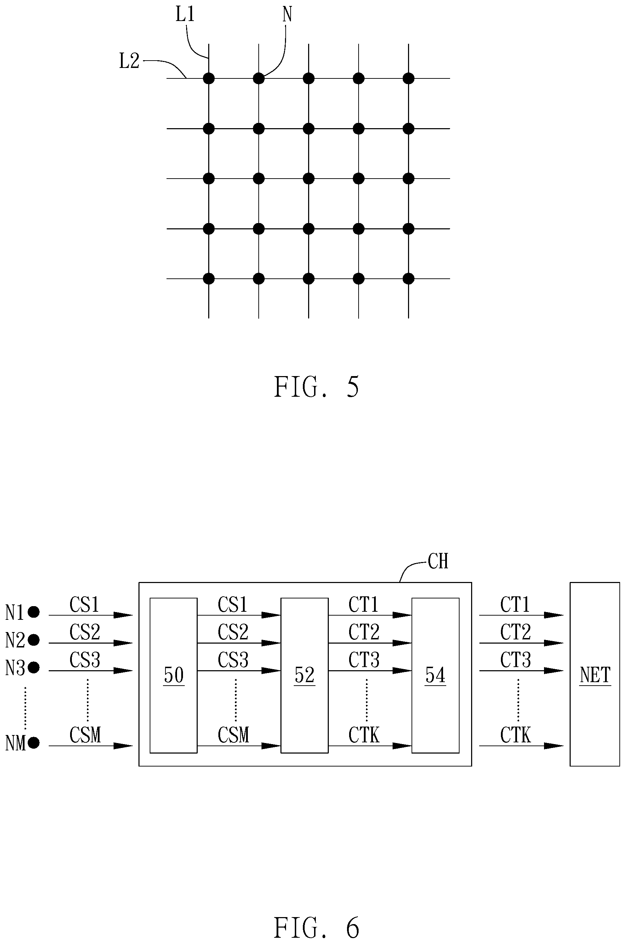

In the actual application, as shown in FIG. 5, the capacitance pressure detection insole 2 may be formed by combining a capacitance line L1 and a conductive line L2 respectively under the thermoplastic polyester elastomer layer TP, and the plurality of capacitance sensing nodes N may be located at the crossing points of the capacitance line L1 and the conductive line L2, but not limited thereto.

It should be noted that, on the surface of the capacitance line L1, two electro coatings are formed, and a capacitance is formed when electric charges are distributed between the two electro coatings. When the capacitance pressure detection insole 2 hasn't received pressure applied by the user's foot FT, the distribution of the electric charge located between the two charged coating is more dense, that is the density of the electric charge is higher; when the capacitance pressure detection insole 2 receives pressure applied by the user's foot FT, the pressure will squash the capacitance line L1, resulting in a more dispersed distribution of the electric charge located between the two charged coatings. If the distance between the two charged coatings is not altered, the capacitance will be altered because of the lower density of the electric charge, and each of the plurality of capacitance sensing nodes N located at the crossing point of the capacitance line L1 and the conductive line L2 respectively senses the plurality of capacitance variations.

For example, as shown in FIG. 4, when the user's foot FT steps on the capacitance pressure detection insole 2, because locations of the plurality of capacitance sensing nodes N1, N2, N3,...of the capacitance pressure detection insole 2 respectively correspond to a plurality of detection points P1, P2, P3, ... of the foot FT, the plurality of capacitance sensing nodes N1, N2, N3,...of the capacitance pressure detection insole 2 will correspondingly sense the capacitance variations corresponding to the plurality of detection points P1, P2, P3,... of the foot FT, and the capacitance variations are transmitted to the operational chip CH .

Please refer to FIG. 6, the operational chip CH at least includes a receiving unit 50, a numerical conversion unit 52 and an output unit 54. Wherein, the numerical conversion unit 52 is coupled between the receiving unit 50 and the output unit 54. When the receiving unit 50 respectively receives the M number of capacitance variations CS1 to CSM from the M number of capacitance sensing nodes N1 to NM, the receiving unit 50 will transmit the M number of capacitance variations CS1 to CSM to the numerical conversion unit 52, and the numerical conversion unit 52 uses a numerical conversion mechanism to conduct a numerical conversion process to the M number of capacitance variations CS1 to CSM to generate K number of numerical converted data CT1 to CTK, wherein both M and K are positive integers and K is smaller than M, so that the data amount of the K number of numerical converted data generated by the numerical conversion unit 52 will be smaller than that of the M number of capacitance variances received by the numerical conversion unit 52 to achieve the effects of decreasing data amount.

It should be noted that, the numerical conversion mechanism used by the numerical conversion unit 52 may be a Fourier transform mechanism or a Laplace transform mechanism, but not limited thereto. Next, the Fourier transform and the Laplace transform are respectively described:

Fourier transform is a linear integral transform, often applied in the field such as physics or engineering to transform signals between time domain (or airspace) and frequency domain. In signal processing, the typical use of the Fourier transform is to decompose signals into amplitude components and frequency components. It is named after a French scholar Jean Baptiste Joseph Fourier because he was the first one who suggested the fundamental concept systematically.

Laplace transform is a linear integral transform often used in applied mathematics to transform a function with a real number argument (larger or equal to 0) to a function whose argument is a complex number. It is named after a French astronomer and mathematician Pierre-Simon marquis de Laplace because he was the first one who used it in his study of probability theory.

Laplace transform is related to Fourier transform, but the difference between them is: Fourier transform shows a function or a signal as superpositions of many sine waves, but Laplace transform shows a function as superpositions of many matrixes. In physics and engineering, Laplace transform is often used to analyze a linear time-invariant system and may conduct transformation between time domains and frequency domains, wherein in the time domain, both input and output are functions of time (the unit is second), and in the frequency domain, both input and output are functions of complex angular frequency (the unit is radian/second).

For example, if M is 1000, that is, the numerical conversion unit 52 receives 1000 capacitance variations CS1 to CS1000 respectively corresponding to 1000 detection points P1 to P1000 of the foot FT, the numerical conversion unit 52 may immediately conduct a sampling process to the 1000 capacitance variations CS1 to CS1000 through Fourier transform mechanism or Laplace transform mechanism, sampling a capacitance variation to generate 100 numerical converted data CT1 to CT100 (that is, K is 100) by every 10 detection points. Since the data amount after the numerical conversion processes is only 1/10 of the original data, the total data amount may be effectively decreased, which makes the operational chip CH capable of running processes of storing and processing.

Other than this, the operational chip CH may also decrease the data amount by a selection method. For example, when the operational chip CH receives 1000 capacitance variations CS1 to CS1000 (that is, M is 1000) respectively corresponding to100 detection points P1 to P1000 of the foot FT, the operational chip CH may compare the 1000 capacitance variations CS1 to CS1000 with a default value. If only 200 capacitance variations are larger than the default value among the 1000 capacitance variations CS1 to CS1000, it means that the other 800 capacitance variations are very small and should be ignored, and the operational chip CH may immediately generate the numerical converted data C1 to C200 (that is, K is 200) according to the 200 capacitance variations larger than the default value.

It should be noted that, the advantage of this method is to decrease the data amount effectively. Since the amount of unstored capacitance variations are very small, it means that the pressure distribution at the detection point of the foot FT has no clear variations, and may therefore be ignored. They will also not effect the subsequent determinations of the sport physiological status information of the foot.

After the numerical conversion unit 52 generates the K number of numerical converted data CT1 to CTK, the operational chip CH may store the K number of numerical converted data CT1 to CTK, or, based on an analysis of the K numerical converted data CT1 to CTK, determine the sport physiological status information of the foot of the user.

It should be noted that, after the numerical conversion unit 52 generates the numerical converted data, in the subsequent application process, one may first conduct a reverse conversion of the numerical converted data to revert the data back to the original detection data before data processing, or use the data sheet to find the original data corresponding to the K numerical converted data CT1 to CTK. There is no specific limitation.

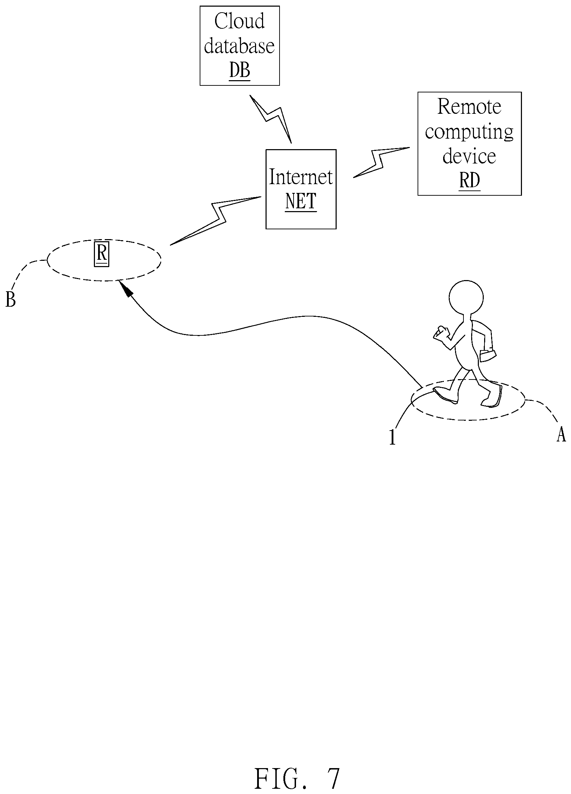

As shown in FIG. 7, in another embodiment, the capacitance variations of the operational chip CH may be transmitted to a cloud database DB and/or a remote computing device RD through other device. In particular, in the present embodiment, the operational chip CH may transmit the information of the capacitance variations to a cloud database DB or a remote computing device RD through an internet NET when it is near a data reading device R. In an actual application, as shown in FIG.4 to FIG.7, the operational chip CH may be connected to a cloud database DB through the internet NET, and may upload pressure distribution information corresponding to the plurality of detection points P1, P2, P3, of the foot FT and/or information such as the sport physiological status information of the user's foot FT to the cloud database DB, as a reference for conducting the other subsequent applications.

For example, the user may conduct a sports activity from a position A to a position B which has a data reading device R. In the present embodiment, the data reading device R may be an internet of Things (IOT) device connected to the internet NET. If the exercise conducted by the user between the position A and the position B is "running", in an embodiment, when the user approaches the data reading device R in the position B, the data reading device R may automatically obtain data of the capacitance variations generated relative to the "running" exercise from the position A to the position B (that is, information such as the sport physiological status information of the foot FT between the position A and the position B) from the operational chip CH .

In an embodiment, as shown in FIG.7, an insole maker may obtain the sport physiological status information of the user's foot FT through the cloud database DB and accordingly determine what the user's foot problem is. For example, the insole maker may obtain sport physiological status information or data of the user from the cloud database DB on the internet through the remote computing device RD.

Based on the sport physiological information or data of the foot FT obtained by the insole maker, the insole maker may reconstitute or simulate the status of the user's foot in exercise, and determine whether the user's foot is of an abnormal status and whether a correction insole is required.

In particular, the user's foot will actually apply pressure on the insole when exercising. Therefore, an average pressure profile and an average pressure force of a normal foot may be obtained by gathering foot data of a plurality of different people. The insole maker may refer to such information as the average pressure profile to determine whether the sport physiological status information of the user's foot pertains to normal or abnormal information. For example, if the ankle of the user has the issues of out-toeing or flat foot, the foot pressure profile according to the sport physiological status information of the foot generated during exercise will be different from the normal shape. In this situation, the insole maker may produce customized correction insoles for the user, and dispose it in the shoes. After the user walks or runs wearing the shoes with the customized correction insole for a period of time, the user's foot problem may be improved significantly.

In addition, the user may also operate an application (APP) of a Mobile Device MB (such as a smartphone) to connect to the operational chip CH or the cloud database DB on the internet NET, so that the sport physiological status information of the foot FT of the user A may be obtained at any time.

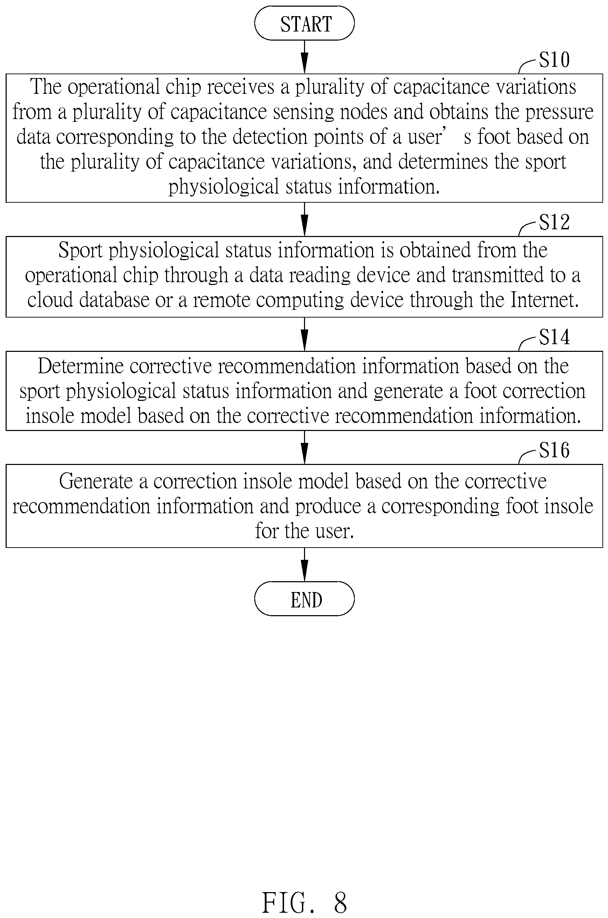

Please refer to FIG. 8. FIG. 8 is a flow chart of a product customization method of the present disclosure. According to the product customization method in the present disclosure, such method may include the following steps:

Step S10: The operational chip receives a plurality of capacitance variations from a plurality of capacitance sensing nodes and obtains the pressure data corresponding to the detection points of a user's foot based on the plurality of capacitance variations, and determines the sport physiological status information.

In particular, at least one of the capacitance pressure detection insoles will be used in the product customization method of the present disclosure. The insole may be disposed in a shoe, and may include an operational chip CH and a plurality of capacitance sensing nodes. As shown in FIG. 2 to FIG. 5, the operational chip CH may be embedded into the capacitance pressure detection insole 2. When the user wears the shoes and begins to run, the user's foot FT will apply pressure on the capacitance pressure detection insole 2. As shown in FIG. 4 to FIG. 5, when the foot FT applies pressure on the capacitance sensing nodes N of the capacitance detection insole 2, the capacitance sensing nodes N which are stepped on will generate different capacitances as opposed to the capacitance sensing nodes not stepped on. The operational chip CH will receive a plurality of capacitance variations and obtain the pressure data corresponding to the detection points of the user's foot based on the capacitance variations.

Step S12: Sport physiological status information is obtained from the operational chip through a data reading device and transmitted to a cloud database or a remote computing device through the internet. As shown in FIG. 3 to FIG. 6, the data reading device R may be an internet of Things (IOT) device or other devices that may connect to the operational chip CH to receive sport physiological status information. In an embodiment, the data reading device R may have the function of Bluetooth or WiFi (Wireless communication), but not limited thereto. In another embodiment, the data reading device R may also connect to the operational chip CH through a wired method to receive sport physiological status information. After receiving sport physiological status information, the data reading device R may transmit the sport physiological status information to the cloud database DB and/ or the remote computing device RD through the internet NET.

Step S14: Determine corrective recommendation information based on the sport physiological status information and generate a foot correction insole model based on the corrective recommendation information. In the present embodiment, if the remote computing device RD hasn't received the sport physiological status information, the remote computing device RD may connect to the cloud database DB through the internet NET to check whether there is a new sport physiological status information or not. In that situation, the remote computing device RD may compare the sport physiological status information with the average value of normal feet to decide whether the sport physiological status is normal or abnormal. When the remote computing device RD determines that the sport physiological status is abnormal, the remote computing device RD may analyze the abnormal information to generate a corrective recommendation.

Step S16: Generate a correction insole model based on the corrective recommendation information and produce a corresponding foot insole for the user. In particular, the remote computing device RD may generate the corresponding correction insole model based on the corrective recommendation information. The model is used to be compared to the portion required to be corrected or adjusted so that the corresponding correction insole may be produced. In the present embodiment, through producing the correction insole, customized insoles may be given to the user to wear.

The abovementioned descriptions of the preferred embodiments merely describe the technical features and the essences of the present disclosure more clearly, the preferred embodiments are not intended to limit the scope of the present disclosure. Conversely, various alternations and equivalents are aimed to be viewed as being embraced by the scope of the claims of the present disclosure.

## Claims

1. A product customization method based on a result of detected pressure, comprising:
receiving capacitance variations from a plurality of capacitance sensing nodes to obtain pressure data corresponding to detection points of a user's foot based on the capacitance variations and to determine sport physiological status informaiton of the foot by an operational chip of a capacitance pressure detection insole;
receiving and transmitting the sports physiological status information by a data reading device to a cloud database or a remote computing device through a network;
determining corrective recommendation information from the sport physiological status information, and generating a foot correction insole model based on the corrective recommendation information; and
providing an insole corresponding to the foot correction insole model.

2. The method of claim 1, wherein the step of determining the sport physiological status informaiton further comprises:
sensing capacitance variations corresponding to the detection points of the foot by the capacitance sensing nodes when the capacitance pressure detection insole receives a pressure applied by the foot.

3. The method of claim 2, further comprising:
receiving the capacitance variations from the plurality of capacitance sensing nodes to obtain pressure distribution informaiton corresponding to the detection points of the foot based on the capacitance variations and to determine the sport physiological status informaiton of the foot by the operational chip.

4. The method of claim 3, wherein the operational chip comprises a receiving unit and a numerical conversion unit, and the step of obtaining the pressure data further comprises:
receiving M number of original detection data by the receiving unit, wherein M is a positive integer; and
conducting a numerical conversion process to the M number of original detection data by the numerical conversion unit to generate K number of numerical converted data,
wherein K is a positive integer and smaller than M, so that the data amount of the K number of numerical converted data is smaller than that of the M number of original detection data.

5. The method of claim 4, further comprising:
using a numerical conversion mechanism to conduct the numerical conversion process to the M number of original detection data by the numerical conversion unit.

6. The method of claim 5, wherein the numerical conversion mechanism is a Fourier transform mechanism or a Laplace transform mechanism.

7. The method of claim 4, further comprising:
outputting the K number of numerical conversion data to the internet; and
connecting the cloud database or the remote computing device through the internet.

8. The method of claim 4, further comprising:
determining the sport physiological status informaiton of the foot according to the K number of numerical converted data; and
transmitting the sport physiological status information to the internet.

9. The method of claim 1, wherein the operational chip is a Bluetooth chip embedded in the capacitive pressure detection insole, and the step obtaining the sport physiological status information from the operational chip by the reading device further comprises:
when the operational chip is located near the data reading device, the operational chip and the data reading device are communicated with each other by Bluetooth communication protocol.

10. The method of claim 1, wherein the step of determining the corrective recommendation information from the sport physiological status information further comprises:
comparing the sport physiological status informaiton with an average value of normal feet; and
analyzing differences between the sport physiological status information and the average value according to the comparison result to generate the corrective recommendation information.

11. The method of claim 10, further comprising:
determining a distribution profile of pressure applied to the insole by the foot based on the sport physiologucal status information; and comparing the distribution profile of pressure with the average value of the normal feet.

12. The method of claim 1, wherein the data reading device comprises a device including internet of things, Bluetooth, or wireless network.
